Europäisches Patentamt

European Patent Office

Office européen des brevets

(11) Veröffentlichungsnummer: **0 303 155**
**A2**

(12) **EUROPÄISCHE PATENTANMELDUNG**

(21) Anmeldenummer: **88112521.5**

(22) Anmeldetag: **02.08.88**

(51) Int. Cl.⁴: **C12Q 1/68**

(30) Priorität: **13.08.87 DE 3726934**

(43) Veröffentlichungstag der Anmeldung:
**15.02.89 Patentblatt 89/07**

(84) Benannte Vertragsstaaten:
**BE CH DE FR GB IT LI NL**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Reckmann, Bernd, Dr.**
**Freiligrathstrasse 7**
**D-6104 Seeheim(DE)**
Erfinder: **Rieke, Erwin, Dr.**
**Hermannstrasse 12**
**D-6104 Seeheim(DE)**

(54) **Verfahren zum Nachweis von Nukleinsäure-Sequenzen.**

(57) Die Erfindung betrifft ein Verfahren zur Bestimmung von Nukleinsäure-Sequenzen mittels Nachweis der RNA durch Sonden, bei dem die die nachzuweisende Nukleinsäure-Sequenz enthaltende DNA spezifisch durch Restriktionsenzyme fragmentiert, die Spaltprodukte mit einer anderen, eine spezifische Promotor-Sequenz enthaltende DNA ligiert und die jeweils resultierende DNA mittels spezifischer RNA-Polymerase in RNA umgeschrieben und amplifiziert wird.

EP 0 303 155 A2

## Verfahren zum Nachweis von Nukleinsäuresequenzen

Die Erfindung betrifft ein neues Verfahren zur Bestimmung von Nukleinsäuresequenzen auf RNA-Ebene unter Verwendung spezifisch fragmentierender Restriktionsenzyme und RNA-Polymerase-Promotoren.

Bestimmungen von Nukleinsäuren in biologischen Proben erlangen mit zunehmender Bedeutung der Molekularbiologie und der hieraus entwickelten Biotechnologie, insbesondere auch im medizinisch-diagnostischen Bereich, einen stetig wachsenden Stellenwert. So läßt sich beispielsweise die Anwesenheit einer bestimmten ViruS-DNA in einer Probe durch Nachweis ihrer spezifischen Nukleinsäure-(Teil)sequenz feststellen. Mit wachsender Bedeutung nehmen aber auch die Anforderungen, die an derartige Nachweise gestellt werden, zu.

Nukleinsäurebestimmungen sind prinzipiell bekannt. Eine der gängigsten Standardmethoden beschäftigt sich damit, die zu bestimmende Nukleinsäure auf der Oberfläche einer festen Matrix, wie z.B. Nitrozellulose, zu immobilisieren und sie mit einer meist mit radioaktiven Phosphor markierten Polynukleotidsonde, deren Sequenz komplementär zu der zu bestimmenden Nukleinsäuresequenz sein muß, zu hybridisieren (z.B. Southern, J. Mol. Biol. 98, 503-517, (1975); Casey u. Davidson, Nucl. Acids Res. 4, 1539-1552, 1977). Der Einsatz von radioaktiven Substanzen ist aber aus Sicherheitsgründen oft problematisch. Radioaktiver Phosphor weist zudem den Nachteil auf, daß er aufgrund seiner sehr kurzen Halbwertszeit nicht länger gelagert werden kann und so oftmals für viele Anwendungen, insbesondere routinemäßige Serienuntersuchungen, ungeeignet ist.

Um die mit dem Einsatz von radioaktiven Isotopen verbundenen Nachteile zu vermeiden, wurden Bestimmungsmethoden entwickelt, die ohne radioaktive Marker arbeiten. So ist aus der EP 0063879 ein Verfahren bekannt, bei dem die Hybridisierung der zu bestimmenden Nukleinsäuresequenz mit einer Polynukleotidsonde, die kovalent gebundenes Biotin an einem Pyrimidin- oder Purinring enthält, durchgeführt wird. Aufgrund der Anlagerung eines Avidin-Enzymkomplexes an das Biotin kann der Nachweis über eine Enzymreaktion erbracht werden. Aus der EP 0151492 ist ferner bekannt, daß das Signal der Hybridisierung durch Einsatz von Streptavidin-Biotin-Enzymkomplexen oder Lectin-biotinylierten Dextran-Lectin-Enzymkomplexen verstärkt werden kann. Ein weiterer Versuch die Sensitivität des Nachweises zu erhöhen besteht darin, die zu hybridisierende Polynukleotid-Sonde mit einem den Marker tragenden Homopolymerschwanz zu versehen, an den weitere Markerstränge gebunden werden können (EP 0204510). All diese Verfahren haben den Nachteil, daß die darin verwendeten Verstärkerlabels und Polynukleotidsonden oft stark unspezifisch mit Komponenten aus der biologischen Probe reagieren oder zu wenige spezifische Komplexe bilden, so daß die erhöhten Anforderungen an Sensitivität nicht erreicht werden, um Hybridisierungsassays zum Nachweis von z.B. Pathogenen in klinischen Proben einzusetzen. Größere Empfindlichkeit scheint man zu erreichen, wenn nicht das Markersignal, sondern die nachzuweisende DNA amplifiziert wird. So beschreibt die EP 0201184 ein Verfahren, bei dem doppelsträngige DNA durch Denaturierung in die Einzelstränge zerlegt und darauf ein spezifischer Primer hybridisiert wird.

In einem mehrfach durchlaufenen Kreisprozess dienen die jeweiligen Einzelstränge als Template für die Primerextension. Dieses Verfahren ist aber wegen der häufigen Denaturierungs-, Hybridisierungs- und Polymerisationsschritte, wobei große Mengen an Polymerase verbraucht werden und überdies von besonders hoch aufgereinigter DNA ausgegangen werden muß, sehr aufwendig und somit zeit- und kostenintensiv.

Es bestand also die Aufgabe, ein neues Verfahren zur Bestimmung von Nukleinsäuresequenzen zu finden, das die geschilderten Nachteile der bislang bekannten Verfahren eliminiert, also von hoher Sensitivität bei gleichzeitiger einfacher Handhabung und hoher Zuverlässigkeit ist.

Überraschend wurde gefunden, daß ein sehr spezifischer und sehr sensitiver Nachweis einer zu bestimmenden Nukleinsäuresequenz dadurch gelingt, daß diese Sequenz vorzugsweise mit bestimmten Restriktionsenzymen spezifisch geschnitten, in diese Schnittstelle ein RNA-Polymerase-Promotor kloniert und anschließend die durch Transkription bzw. Amplifikation gebildete RNA in bekannter Weise nachgewiesen wird.

Gegenstand der Erfindung ist somit ein Verfahren zur Bestimmung von Nukleinsäuresequenzen mittels Nachweis der RNA durch Sonden, dadurch gekennzeichnet, daß man die Nukleinsäuresequenzen spezifisch fragmentiert, die Spaltprodukte mit einer DNA-Sequenz, enthaltend eine spezifische Promotorsequenz und überstehende, zum Spaltprodukt komplementäre Enden, ligiert und die zum Nachweis erforderliche RNA durch Transkription und Amplifikation mittels einer promotor-spezifischen Polymerase herstellt.

Gegenstand der Erfindung ist ferner ein Mittel zur Bestimmung von Nukleinsäuresequenzen mittels Nachweis der RNA durch Sonden, dadurch gekennzeichnet, daß es neben Nachweisreagenzien eine Restriktionsendonuklease, die die zu bestimmenden Nukleinsäuresequenzen nach mehreren beliebigen Nukleotiden innerhalb oder außerhalb einer spezifischen Erkennungssequenz schneidet, eine eine spezifische Promotor-Sequenz enthaltende DNA-Sequenz mit zum Spaltprodukt komplementären Enden, eine Ligase, die die Ligation der DNA-Sequenz mit den Spaltprodukten katalysiert, eine promotorspezifische Polymerase, welche die Transkription und Amplifikation der RNA bewirkt und ggf. eine Polynukleotidsonde zur Hybridisierung enthält.

Gegenwärtig kennt man über 600 verschiedenen Restriktionsenzyme (Kessler, Hölthe (1986), Gene 47, 1-153). Bei dem erfindungsgemäßen Verfahren sind dabei jene Restriktionsendonukleasen bevorzugt, die entweder innerhalb spezifischer Erkennungssequenzen nach mehreren beliebigen internen Nukleotiden spalten oder die Restriktion mehrerer beliebiger Nukleotide nach der spezifischen Erkennungssequenz durchführen, so daß Bruchstücke mit überlappenden Enden entstehen. Bevorzugt sind demnach Restriktionsendonukleasen mit einer Erkennungssequenz
vom Typ A: $(X)_n (N)_m (X)_o$ oder
vom Typ B: $(X)_n (N)_m$
$(Y)_n (N)_p$

worin X    ein bestimmtes, für die Erkennungssequenz spezifisches Nukleotid,
N    ein beliebiges, unspezifisches Nukleotid
Y    ein für die Erkennungssequenz spezifisches und zu X komplementäres Nukleotid
n,o    ganze Zahlen zwischen 2 und 8 und
m,p    eine ganze Zahl zwischen 4 und 20
bedeutet.

Die bevorzugten Enzyme entsprechen den Restriktionsendonukleasen der Klassen II und II-S. Beispiele für Enzyme der Klasse II sind: Sec I, Nta I, Tte I, Ttr I, Dra III, Asp 700, Xnm I, Pfl UI, Bgl I, Hgi EII, Bss GI, Bst TI, Bst XI oder Sfi I. Besonders bevorzugt sind hierunter Bgl I, Bst XI oder Sfi I. Beispiele für Enzyme der Klasse II-S sind: Bbv I, Bbv II, Bin I, Bsp UI, Eco 31I, Fok I, Hga I, Hph I, Ubo II, Sta NI, Tag II oder Tth 111 II. Besonders bevorzugt hierunter ist Fok I. Die meisten der aufgeführten Enzyme, insbesondere die besonders bevorzugten, sind im Handel erhältlich.

Besonders vorteilhaft für den Nachweis einer Nukleinsäuresequenz ist es, wenn das Enzym eine lange Erkennungssequenz aufweist und stark überlappende Enden beim Schnitt produziert. Eine Erkennungssequenz von sechs Nukleotiden bedeutet, daß diese Sequenz, entsprechend der sie vertretenden Basen Adenin, Thymin, Guanin und Cytosin, alle $4^6 = 4096$ Nukleotide vorkommt und eine von acht bzw. 11 Nukleotiden, daß sie alle $4^8 = 65536$ bzw. $4^{11} = 4,2 \times 10^6$ Basen auftritt. Schneidet man beispielsweise das E. coli-Genom (ca. $3 \times 10^6$ Nukleotide) mit der bevorzugten Restriktionsendonuklease Sfi I (GGCC(N₄)/NGGCC), so entstehen, statistisch gesehen, 46 Fragmente, die spezifisch überlappende, terminale Trinukleotidsequenzen aufweisen. Die statistische Wahrscheinlichkeit ein ganz bestimmtes Trinukleotid anzutreffen ist somit kleiner als 1. Schneidet man das E. coli-Genom z.B. mit Fok I, so entstehen statistisch gesehen sogar nur 3 Fragmente mit spezifischen Tetranukleotidsequenzen. Diese hohe Spezifität wird nun beim erfindungsgemäßen Verfahren zum Nachweis von spezifischen Nukleinsäuresequenzen ausgenutzt.

Dazu ligiert man mittels Ligase in an sich bekannter Weise ein DNA-Sequenz, die eine komplementäre überlappende Nukleotidsequenz enthält, mit den durch eine spezifische Restriktionsendonuklease erzeugten und die nachzuweisende Nukleinsäure-Sequenz enthaltenden DNA-Fragmenten. Die zur Ligierung erforderliche überlappende Nukleotidsequenz kann durch entsprechende Nukleotidsynthese nach Standardmethoden variiert werden. Erfindungsgemäß enthält die DNA-Sequenz einen Promotorbereich, der eine RNA-Polymerase zu binden vermag. Dadurch kann nach dem erfindungsgemäßen Verfahren das gesamte nach der Ligation entstandene und die nachzuweisende Nukleinsäuresequenz enthaltende jeweilige DNA-Fragment in RNA umgeschrieben und amplifiziert werden. Ist der Promotor spezifisch genug, so wird erfindungsgemäß nur dann RNA gebildet, wenn die nachzuweisende Nukleinsäure- bzw. DNA-Sequenz auch tatsächlich in der Probe vorhanden ist. Dies bedeutet, daß normalerweise keine Fremd-DNA transkribiert wird.

Als Promotoren/Polymerasen eignen sich alle bekannten spezifisch wirksamen Promotoren/Polymerasen, beispielsweise E. coli RNA Polymerase, SP 6-Polymerase, T7-Polymerase oder T3-Polymerase.

Prinzipiell können auch regulierbare, z.B. temperatursensitive Promotoren wie z.B. der bekannte $\lambda$-$P_L$-Promotor, eingesetzt werden. Bevorzugt sind virale Promotoren und Polymerasen, insbesondere der SP6- oder der T7-Promotor. Beide sind sehr starke, für die entsprechende Polymerase absolut spezifische Promotoren, die zu einer effizienten Transkription führen (Chamberlain et al., Methods in Enzymology, 101, 540-568 (1983), Dunn, Studier, J. Mol. Biol., 166, 477 (1983); Kassavetis et al., J. Biol. Chem., 257, 5779 (1982)) und zudem im Handel erhältlich sind. Besonders geeignet ist das T7-Promotor/Polymerase-System.

Die amplifizierte RNA läßt sich in an sich bekannter Weise durch Einführung von Sondenmolekülen nachweisen. Als Sonden eignen sich radioaktive Label oder nicht radioaktive Marker. Vorzugsweise werden nicht radioaktive Sonden eingesetzt. Der radioaktive Nachweis erfolgt beispielsweise durch Filmschwärzung oder Szintillationszählung nach Umsetzung von RNA-Nukleotiden mit vorzugsweise $\alpha$-$^{32}$P-ATP. Bei dem bevorzugten nicht radioaktiven Nachweis führt man eine Umsetzung von RNA-Nukleotiden mit vorzugsweise Biotin-11-UTP durch, wodurch die RNA biotinyliert wird. Der eigentliche Nachweis erfolgt dann bevorzugt über eine Enzymreaktion, beispielsweise durch Bildung eines Avidin-Biotin-, eines Streptavidin-Biotin oder eines Lectin-biotinylierten-Dextran-Lectin-Enzymkomplexes nach bekannter Methodik (z.B. Langer et al., Proc. Natl. Acad. Sci. USA, 78, 6633 (1981); Brigati et al., Virology, 126, 32 (1983)). Als Enzym wird vorzugsweise Phosphatase verwendet, deren Aktivität sich durch eine empfindliche Farbreaktion nachweisen läßt. Die Einführung der Sonden erfolgt nach dem erfindungsgemäßen Verfahren entweder direkt während der in vitro-Transkription der DNA-Sequenz oder aber nach erfolgter Bildung der RNA durch Hybridisierung mit einer ggf. radioaktiv markierten Polynukleotidsonde, deren Sequenz komplementär zu dieser RNA ist und einen Teil der nachzuweisenden DNA entspricht. Erfindungsgemäß wird die Markierung auf Transkriptionsebene bevorzugt, da man hierdurch den Hybridisierungsschritt einspart. Die zusätzliche Hybridisierung ist jedoch dann von Vorteil, wenn die zu bestimmende Probe einerseits große Mengen fremde komplexe Nukleinsäuresequenzen enthält, andererseits nur Restriktionsenzyme mit kürzeren Erkennungssequenzen und kürzeren überlappenden Spaltenden zur Verfügung stehen, so daß die erforderliche Spezifität nicht ausreicht, und auch RNA von Fremd-DNA-Fragmenten aufgrund gleicher Sequenz der überlappenden Enden transkribiert wird. Durch die zusätzliche Hybridisierung mit spezifischen markierten Polynukleotiden wird hingegen auch in diesen Fällen die erfindungsgemäß hohe Spezifität bei immer noch deutlich höherer Sensitivität als bislang bekannt, erreicht.

Die dem erfindungsgemäßen Verfahren zugrundeliegende experimentelle Durchführung entspricht ausnahmslos den auf diesem Gebiet gängigen bekannten Standardmethoden. Eine allgemeine Beschreibung findet sich z.B. in Maniatis et al., In: Molecular Cloning, 269, Cold Spring Harbor, NY (1982). Synthesevorschriften für Oligonukleotide zur Darstellung der Promotor-haltigen DNA-Sequenzen sind beispielsweise bei Gait, Oligonucleotide Synthesis, IRL Press, Oxford (1984) zu entnehmen. Die Ligation von DNA-Fragmenten ist z.B. bei Weiss, et al., J. Biol. Chem., 243, 4543 (1968) beschrieben. Bedingungen für bevorzugte Restriktionsenzyme und Polymerasen sind unter anderem aus Angaben der Hersteller zu ersehen. Weitere Angaben sowie die detaillierte Beschreibung des bevorzugt durchgeführten Verfahrens sind in den unten stehenden Beispielen wiedergegeben.

Das erfindungsgemäße Verfahren bietet eine Vielzahl offensichtlicher Vorteile gegenüber den herkömmlichen Methoden, insbesondere gegenüber den bekannten Hybridisierungstechniken.

So fallen alle normalerweise für eine Hybridisierung notwendigen drastischen und zeitraubenden Schritte wie beispielsweise Immobilisierung der Nukleinsäure, Denaturierung der DNA durch Hitze oder Lauge, oft langwierige Hybridisierung bei hohen Temperaturen oder in Anwesenheit von Formamid und Waschschritte zur Entfernung nicht hybridisierter Probe vollständig weg. Dadurch ist der Nachweis einer Nukleinsäuresequenz mit wesentlich weniger Arbeitsschritten verbunden.

Darüberhinaus liefert das erfindungsgemäße Verfahren eine sehr hohe Spezifität bezüglich einer bestimmten nachzuweisenden Nukleinsäure-Sequenz. Prinzipiell können nach diesem Verfahren auch Bestimmungen in Proben mit komplexen, fremden Nukleinsäure-Anteilen durchgeführt werden, wenn entsprechende Restriktionsenzyme mit sehr langen Erkennungs- und Überlappungssequenzen bereitgestellt werden können.

Letztlich wird durch das erfindungsgemäße Verfahren die Sensitivität des Nachweises gegenüber den herkömmlichen Methoden aufgrund des starken Amplifikationseffektes der RNA-Polymerase/Promotor-Systems deutlich gesteigert. So liegt die routinemäßig erreichbare Nachweisgrenze z.B. mit Biotin-Sonden nach dem neuen Verfahren zwischen 10 und 100 fg, vorzugsweise zwischen 30 und 50 fg. Dies ist gegenüber den bekannten Verfahren eine Steigerung um den Faktor 100 bis 1000.

Zur Durchführung des erfindungsgemäßen Verfahrens, insbesondere auch in Kliniken, eignen sich auch Testpackungen, welche entsprechend der jeweiligen Anwendung in qualitativ und quantitativ aufeinander abgestimmter Form eine Restriktionsenzym, eine eine spezifische Promotor-Sequenz enthaltende DNA-Sequenz, ein Ligations- und RNA-Polymerisations-Enzym, Nachweisreagenzien, Puffersysteme und ggf. eine zur Hybridisierung geeignete Polynukleotidsonde enthalten. Als Nachweisreagenzien können beispielsweise Biotin-11-UTP, Streptavidin-biotinylierter alkalischer Phosphatasekomplex sowie ein für den Phosphatnachweis spezifisches Farbreagenz in aufeinander abgestimmter Form dienen. Erfolgt der Nachweis über radioaktive Messung, dient als Nachweisreagenz ein radioaktiv markiertes Derivat, beispielsweise $\alpha$-$^{32}$P-ATP, welches mit der transkribierten RNA zur Reaktion gebracht wird.

Vor- und nachstehend haben die genannten Abkürzungen folgende Bedeutung.

A    Adenin

C     Cytsin
G     Guanin
T     Thymin
DNA     Desoxyribonukleinsäure
RNA     Ribonukleinsäure
DTT     DL-Dithiothreitol
EDTA     Ethylendiamintetraessigsäure
BSA     Rinderserumalbumin
ATP     Adenosintriphosphat
GTP     Guanosintriphosphat
UTP     Uridintriphosphat
CTP     Cytidintriphosphat
SDS     Natriumdodecylsulfat
Tris     Trishydroxymethylaminomethan
PBS     phosphatgepufferte Kochsalzlösung
SSPE     Natriumchlorid-Natriumphosphat-EDTA
SSC     Natriumchlorid-Natriumcitrat

Beispiel 1

Spaltung der die nachzuweisende Nukleinsäuresequenz enthaltenden DNA durch Restriktionsenzyme.

    10 µg doppelsträngige DNA des M13mp18-Virus (7250 Basenpaare) (Messing, 1983, Methods in Enzymology, 101, 20 Academic Press, NY) werden mit 20 Units der Restriktionsendonuklease Fok I bei 30-40 °C, vorzugsweise 36-38 °C für 1 bis 3 Stunden in einem Tris/HCl-Puffer (10 mM, pH = 7,5) inkubiert. Nach vollständiger Spaltung wird der Ansatz in an sich bekannter Weise phenolisiert und die DNA durch Ethanol gefällt.
    Das Restriktionsenzym Fok I spaltet die Sequenz

GGATG (N$_9$) CCTAC (N$_{13}$)
und erzeugt so vier Tetranukleotidsequenzen mit folgender Überlappungssequenz:

5′-TTAA, 5′-AAAA, 5′-TCGC und 5′-GTAG.

Beispiel 2

Ligation von durch Restriktionsenzyme erhaltenen DNA-Fragmenten mit einer Promotor-Sequenz (T7-Promotor).

    Entsprechend den beiden Strängen mit bekannter Sequenz des T7-Promotors (Dunn, Studier; Kassavetis et al., l.c.) werden Oligonukleotide durch chemische Synthese in an sich bekannter Weise (Gait, l.c.) synthetisiert und nach Standardmethoden am 5′-Ende durch käufliche T4-Polynukleotidkinase phosphoryliert. Anschließend werden beide Oligonukleotide im molaren Verhältnis 1:1 durch Erhitzen auf etwa 55 °C und Abkühlung auf Raumtemperatur hybridisiert. Es entsteht ein doppelsträngiges DNA-Fragment mit überlappenden Enden von folgender Struktur:

5′ GCGATTCGAAATTAATACGACTCACTATAGGGAG    -3′
3′-AAGCTTTAATTATGCTGAGTGATATCCCTCCGCT-5′

Zur Ligation werden 5 µg der vier Fok-I-gespaltenen DNA-Fragmente gemäß Beispiel 1 mit etwa dem 10fachen molaren Überschuß des synthetisierten doppelsträngigen, die T7-Promotor-Sequenz enthaltenden Oligonukleotids zusammen mit 10 Units käuflicher T4-DNA-Ligase in einem Tris/HCl-Puffer (50 mM, pH: 7,6, 1 mM ATP, 10 mM MgCl$_2$, 10 mM DTT) für 5-15 Stunden bei ca. 15 °C inkubiert und die DNA wird anschließend mit Ethanol gefällt. Aufgrund der Sequenz der überlappenden Enden hat eine Ligation des synthetisierten doppelsträngigen Oligonukleotids nur mit dem 5′-TCGC-Fragment der M13mp18-DNA stattgefunden.

Beispiel 3

In vitro-Transkription und Amplifikation von RNA

    Die DNA aus den Ligationsansätzen aus Beispiel 2 wird mit käuflicher T7-RNA-Polymerase in vitro transkribiert. Dazu wird je 1 µg der ligierten DNA-Fragmente in 20 µl Reaktionsvolumen mit ca. 40 Units T7-RNA-Polymerase bei 35-38 °C für 2-4 h in einem Inkubationsmedium, enthaltend 40 mM Tris/HCl (pH: 7,2), 6 mM MgCl$_2$, 10 mM Spermidin und je 0,4 mM GTP, UTP, CTP und ATP, inkubiert. Anschließend werden die Reaktionsansätze auf einem 1 %igen Agarose-Gel in an sich bekannter Weise zusammen mit einer Probe vom Ligationsansatz elektrophoretisiert und die Nukleinsäuren durch Färbung mit Ethidiumbromid sichtbar gemacht.
Der Transkriptionsansatz liefert eine starke Bande im Bereich von 500-1000 Nukleotiden Länge, während der Ligationsansatz keine solche Bande auf dem Agarosegel aufweist. Dies beweist, daß ein M13mp18-DNA-Fragment transkribiert wurde.

Beispiel 4

Nachweis durch Bildung Biotin-markierter RNA

Je 1 μg der Ligationsansätze aus Beispiel 2 werden standardgemäß in Anwesenheit von Biotin-11-UTP (Langer et al., l.c.) transkribiert. Dazu werden die ligierten DNA-Fragmente mit ca. 40 Units T7-RNA-Polymerase und 1 mM Biotin-11-UTP anstelle von UTP unter den in Beispiel 3 angegebenen Bedingungen inkubiert. Nicht umgesetztes Biotin-11-UTP wird anschließend durch Chromatographie in an sich bekannter Weise abgetrennt. Das Eluat wird verdünnt und verschiedene Mengen zwischen 1 ng und 1 fg werden auf eine Nylonmembran aufgetragen. Diese wird zunächst zur Vermeidung unspezifischer Bindungen in einer Blockierlösung (1 x PBS, 2 % BSA, 0,05 % Triton X-100, 5mM EDTA) und anschließend in einer Lösung (1 x PBS, 5 mM EDTA, 0,5 % Triton X-100, 0,1 % BSA) eines Streptavidin-biotinylierten alkalischen Phosphatase-komplexes (Brigati et al., l.c.) jeweils 30 bis 60 Minuten bei Raumtemperatur inkubiert. Die Membran wird gewaschen und zur Detektion in einer Lösung von Nitroblautetrazolium (ca. 75 mg/ml) und 5-Brom-4-chlor-3-indolylphosphat (50 mg/ml), 100 mM Tris/HCl (pH: 8,8), 100 mM NaCl und 5 mM MgCl$_2$ bei Raumtemperatur inkubiert, bis deutliche Farbflecken sichtbar werden.

Man erhält eine deutliche Farbreaktion für das M13mp18-DNA-Fragment mit der überlappenden Sequenz 5'-TCGC. Die Menge, die noch zu einer deutlichen Farbreaktion führt, beträgt unter den genannten Bedingungen 20-30 fg.

Beispiel 5

Nachweis von amplifizierter RNA durch Hybridisierung mit komplementärer DNA.

Das durch Oligonukleotidsynthese herstellbare Oligonukleotid 5'-CAGGAAACAGCTATGAC-3', welches einer Sequenz auf der M13mp18-DNA ca. 100 Nukleotide vom Startpunkt der Transkription entspricht, wird durch Einbau von γ-$^{32}$P-ATP mittels T4-Polynukleotidkinase radioaktiv markiert. Die gemäß Beispiel 3 gewonnene RNA sowie unbehandelte M13mp18-DNA werden in verschiedenen Mengen auf eine Nylonmembran fixiert. Letztere wird in einer Lösung, enthaltend 6 x SSC, 5 x Denhardt, 0,5 % SDS und 20 μg/ml Heringssperma-DNA bei 40 bis 50 °C prehybridisiert. Anschließend wird eine Lösung des gelabelten Oligonukleotids zugesetzt und bei 40-50 °C hybridisiert. Nach Waschen wird die Membran mit einem Röntgenfilm bedeckt und 10 bis 20 Stunden bei ca. -20 °C exponiert.

Man erzielt Hybridisierung sowohl von unbehandelter M13mp18-DNA als auch der RNA-Transkripte aus Beispiel 3. Die Nachweisgrenze für die RNA-Transkripte liegt niedriger als für die M13mp18-DNA, jedoch noch zwei- bis dreimal höher als nach herkömmlicher Methodik.

Beispiel 6

Restriktion, Ligation und Transkription von OX 174 Virus-DNA und pBR 322 Plasmid-DNA

Analog Beispiel 1 werden OX 174-DNA (Langer et al., l.c.) und pBR 322-DNA (Bolivar et al., 1977, Gene 2, 95-113) der vollständigen Spaltung mit der Restriktionsendonuklease Fok I unterworfen. Man erhält bei pBR 322 elf und bei OX 174 acht Fragmente mit bekannten Überlappungssequenzen.

Ligation und Transkription erfolgen analog Beispiel 2 und 3.

Auf dem Agarosegel ergibt sich kein Unterschied zwischen dem jeweiligen Ligations- und Transkriptionsansatz. Dies zeigt, daß eine RNA-Bildung für OX 174- und pBR 322-Fragmente unter diesen Bedingungen im Gegensatz zu M13mp18 nicht stattfindet, da die Überlappungssequenz der Promotor-DNA offensichtlich nicht komplementär zu den Überlappungssequenzen der Spaltfragmente ist. Aus diesem Grund erfolgt auch keine Bildung Biotin-markierter RNA gemäß Beispiel 4.

M13mp18-DNA läßt sich somit spezifisch nachweisen.

Beispiel 7

Ligierung und Transkription mit SP6-RNA-Polymerase-Promotor.

Die durch Fok I erzeugten DNA-Fragmente von M13mp18 werden analog Beispiel 2 mit einem doppelsträngigen Fragment mit überlappenden Enden, welches den SP6-Promotor enthält, ligiert und anschließend wie in Beispiel 3 und 4 behandelt und das entsprechende Fragment nachgewiesen, jedoch erfolgt die Transkription mit dem bekannten SP6-Promotor SP6-Polymerase-System.

Man erhält wiederum eine deutliche Farbreaktion für das M13mp18-DNA-Fragment mit der überlappenden Sequenz 5′-TCGC. Die Nachweisgrenze liegt zwischen 40 und 50 fg.

Beispiel 8

Nachweis von OX 174-DNA in Anwesenheit von E. coli-DNA und Kalbsthymus-DNA

Eine Lösung, enthaltend 1 μg OX 174-DNA und je 10 μg E. coli- und Kalbsthymus-DNA, sowie eine Lösung, enthaltend nur je 10 μg E. coli- und Kalbsthymus-DNA werden mit etwa je 10 Units der Restriktionsendonuklease Bst XI in 50 mM Tris/Hcl-Puffer (pH: 7,5) unter üblichen Restriktionsbedingungen versetzt. Anschließend erfolgt in an sich bekannter Weise die Ligation mit 50 ng des 5′-phosphorylierten doppelsträngigen Oligonukleotids mit der Sequenz

5′-
CTCCCTATAGTGAGTCGTATTAATTTCGAACATC-3′

3′-
GTAGGAGGGATATCACTCAGCATAATTAAAGCTT-5′

unter Verwendung von ca. 10 Units bekannter T4-DNA-Ligase. Die Sequenz des Oligonukleotids entspricht dem T7-RNA-Polymerase-Promotor; die überstehenden Enden sind komplementär zu Enden, die bei der Spaltung von OX 174-DNA mit Bst XI entstehen. Das Oligonukleotid wird nach Standardmethoden chemisch synthetisiert (z.B. Gait, l.c.). Die Transkription der beiden Ansätze mit T7-RNA-Polymerase erfolgt analog Beispiel 3.

Man erhält in beiden Ansätzen große Mengen RNA, woraus zu entnehmen ist, daß die den T7-Promotor enthaltende DNA-Sequenz auch in Spaltprodukte der E. coli- und Kalbsthymus-DNA ligiert worden ist.

Zum spezifischen Nachweis von OX 174-DNA werden unterschiedliche Mengen der beiden Ansätze auf Nylonmembran aufgetragen und mit einer durch "nick translation" biotinylierten OX 174-DNA in an sich bekannter Weise hybridisiert. Die Prehybridisierung erfolgt in 50 % Formamid, 5 x SSPE, 5 x Denhardt, 10 % Dextransulfat und 20 μg/ml Heringssperma-DNA für ca. 1 Stunde bei 40-45 °C. Nach mehrfachem Waschen wird die Hybridisierung durch einen Streptavidin-biotinylierten alkalischen Phosphatase-Komplex, wie in Beispiel 4 beschrieben, nachgewiesen.

Im Ansatz mit OX 174-DNA geben 0,5 pg noch ein deutlich sichtbares Signal, während im Ansatz ohne OX 174-DNA keine Farbflecken sichtbar sind. Damit ist OX 174-DNA trotz Anwesenheit großer Überschüsse komplexer Fremd-DNA spezifisch und mit großer Sensitivität nachgewiesen worden.

Ansprüche

1. Verfahren zur Bestimmung von Nukleinsäuresequenzen mittels Nachweis der RNA durch Sonden, dadurch gekennzeichnet, daß man die Nukleinsäuresequenzen spezifisch fragmentiert, die Spaltprodukte mit einer DNA-Sequenz, enthaltend eine spezifische Promotorsequenz und überstehende, zum Spaltprodukt komplementäre Enden, ligiert und die zum Nachweis erforderliche RNA durch Transkription und Amplifikation mittels einer promotor-spezifischen Polymerase herstellt.

2. Verfahren nach Anspruch 1, dadurch gekennzeichnet, daß man die Nukleinsäuresequenzen mit Hilfe von Restriktionsendonukleasen, die die zu bestimmenden Nukleinsäuresequenzen nach mehreren beliebigen Nukleotiden innerhalb oder außerhalb einer spezifischen Erkennungssequenz schneidet, in Teilsequenzen mit überstehenden Enden fragmentiert.

3. Verfahren nach Anspruch 1 und 2, dadurch gekennzeichnet, daß man die Ligation mit einer DNA-Sequenz, welche die Sequenz des T7-Promotors enthält, und die sich anschließende Transkription mit T7-Polymerase durchführt.

4. Mittel zur Bestimmung von Nukleinsäuresequenzen mittels Nachweis der RNA durch Sonden, dadurch gekennzeichnet, daß es neben Nachweisreagenzien eine Restriktionsendonuklease, die die zu bestimmenden Nukleinsäuresequenzen nach mehreren beliebigen Nukleotiden innerhalb oder außerhalb einer spezifischen Erkennungssequenz schneidet, eine eine spezifische Promotor-Sequenz enthaltende DNA-Sequenz mit zum Spaltprodukt komplementären Enden, eine Ligase, die die Ligation der DNA-Sequenz mit den Spaltprodukten katalysiert, eine promotorspezifische Polymerase, welche die Transkription und Amplifikation der RNA bewirkt und ggf. eine Polynukleotidsonde zur Hybridisierung enthält.